# EUROPEAN PATENT APPLICATION

(11) **EP 4 053 098 A1**
(43) Date of publication of application: **07.09.2022**
(21) Application number: 20880470.8
(22) Date of filing: 30.10.2020
(51) Int. Cl.: C07C 67/307, C07C 69/63

(54) **METHOD FOR MANUFACTURING FLUOROACETIC ACID ESTER**

(30) Priority: 31.10.2019 JP 2019198220
(71) Applicant: Daikin Industries, Ltd., Osaka-shi, Osaka 530-8323 (JP)
(72) Inventor: SHIRAI, Atsushi, Osaka-shi, Osaka 530-8323 (JP); ISHIHARA, Sumi, Osaka-shi, Osaka 530-8323 (JP); MATSUURA, Makoto, Osaka-shi, Osaka 530-8323 (JP); KISHIKAWA, Yosuke, Osaka-shi, Osaka 530-8323 (JP)
(74) Representative: Hoffmann Eitle
(86) International application number: PCT/JP2020/040943
(87) International publication number: WO 2021/085631

(57) **Abstract**

An object of the present disclosure is to provide a method for producing a fluoroacetic acid ester with excellent economic efficiency, quality, and productivity.

The object can be solved by a method for producing a compound represented by formula (1): 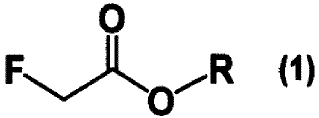 wherein
R is an organic group,
the method comprising:
the step of reacting a compound represented by formula (2): 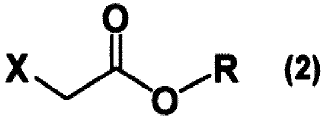 wherein
X is a leaving group other than fluorine, and R is as defined above,
with a compound represented by formula (3):

MFₙ (3)

wherein
M is a cation and n is an integer corresponding to the valence of the cation,
wherein
the compound represented by formula (3) is used in an amount of 0.9 moles or less per mole of the compound represented by formula (2) .

## Description

### Technical Field

The present disclosure relates to a method for producing a fluoroacetic acid ester, and the like.

### Background Art

Fluoroacetic acid esters are effective compounds as starting materials of pharmaceuticals and agrochemicals. As a method for producing a fluoroacetic acid ester, a method for reacting a chloroacetic acid ester with KF in the presence of an additive is known. On the other hand, a method in which an additive is not used is also known. As an example, Non-patent Literature 1 discloses a method comprising reacting a chloroacetic acid ester with 1.2 equivalents of KF, then adding an ether to filtrate an inorganic material, and isolating a target product by distillation. Non-patent Literature 2 discloses a method comprising reacting a chloroacetic acid ester with 1.1 equivalents of KF, then directly subjecting the resultant to distillation to distill an organic material, followed by rectification to isolate a target product.

### Citation List

### Non-patent Literature

NPL 1: Saunders, B.C. et al., Journal of the Chemical Society (1948) pp. 1773-1779
NPL 2: Gryszkiewicz-Trochimowski, E. et al., Rec. Trav. Chim. (1947) 66, p. 413

### Summary of Invention

### Technical Problem

The method using an additive is uneconomical, and has a risk of mixing the additive as an impurity into a target product, which raises quality concerns. On the other hand, in the methods of Non-patent Literature 1 and 2, the resultant is in a wet, crystalline state in a reaction vessel, and efficient stirring cannot be performed. Thus, performing such methods is industrially difficult, resulting in reduced productivity.

An object of the present disclosure is to provide a method for producing a fluoroacetic acid ester with excellent economic efficiency, quality, and productivity; and the like.

The present disclosure includes the following aspects.
[1] A method for producing a compound represented by formula (1): 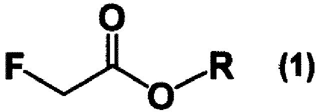 wherein
   R is an organic group,
   the method comprising:
      the step of reacting a compound represented by formula (2): 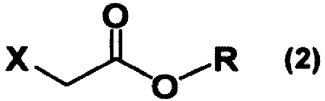 wherein
   X is a leaving group other than fluorine, and R is as defined above,
   with a compound represented by formula (3):

      MFₙ (3)
   wherein
   M is a cation and n is an integer corresponding to the valence of the cation,
   wherein
   the compound represented by formula (3) is used in an amount of 0.9 moles or less per mole of the compound represented by formula (2) .
[2] The production method according to Item 1, wherein the reaction is substantially performed with the compound represented by formula (2) and the compound represented by formula (3) alone.
[3] The production method according to Item 1 or 2, wherein the compound represented by formula (3) is used in an amount of 0.8 moles or less per mole of the compound represented by formula (2) .
[4] The production method according to any one of Items 1 to 3, wherein the compound represented by formula (3) is used in an amount of 0.7 moles or less per mole of the compound represented by formula (2).
[5] The production method according to any one of Items 1 to 4, wherein the compound represented by formula (3) is used in an amount of 0.6 moles or less per mole of the compound represented by formula (2).
[6] The production method according to any one of Items 1 to 5, wherein the reaction is performed at 100 to 250°C.
[7] The production method according to any one of Items 1 to 6, comprising the step of distilling a product obtained by the above step.
[8] The production method according to any one of Items 1 to 7, wherein R is a hydrocarbon group optionally having one or more substituents.
[9] The production method according to any one of Items 1 to 8, wherein R is an alkyl group, a fluoroalkyl group, or an aryl group optionally having one or more substituents.
[10] The production method according to any one of Items 1 to 9, wherein X is halogen other than fluorine, an alkylsulfonyloxy group, a haloalkylsulfonyloxy group, or an arylsulfonyloxy group.
[11] The production method according to any one of Items 1 to 10, wherein X is chlorine or bromine.
[12] The production method according to any one of Items 1 to 11, wherein X is chlorine.
[13] The production method according to any one of Items 1 to 12, wherein M is a hydrogen ion, an alkali metal ion, an alkaline earth metal ion, or an ammonium ion.
[14] The production method according to any one of Items 1 to 13, wherein M is a hydrogen ion or an alkali metal ion.
[15] The production method according to any one of Items 1 to 13, wherein the compound represented by formula (3) is at least one member selected from HF, NaF, KF, CsF, and CaF₂.
[16] The production method according to any one of Items 1 to 15, wherein the compound represented by formula (3) is at least one member selected from KF and CsF.
[17] The production method according to any one of Items 1 to 16, wherein the compound represented by formula (3) has a moisture content of 1.0 mass% or less.
[18] A composition comprising
   a compound represented by formula (1): 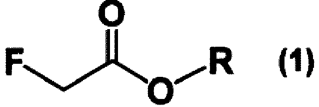 wherein R is an organic group, and
   a compound represented by formula (2): 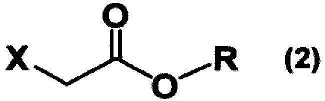
   wherein X is halogen other than fluorine, an alkylsulfonyloxy group, a haloalkylsulfonyloxy group, or an arylsulfonyloxy group, and R is an organic group,
   wherein
   the content of the compound represented by formula (2) is more than 0.001 mass% to 2.5 mass% or less.
[19] A composition comprising
   a compound represented by formula (1): 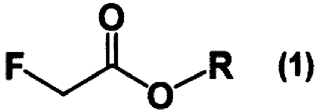 wherein R is an organic group, and
   a compound represented by formula (2): 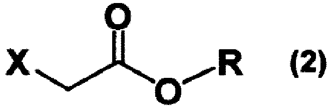
   wherein X is halogen other than fluorine, an alkylsulfonyloxy group, a haloalkylsulfonyloxy group, or an arylsulfonyloxy group, and R is an organic group,
   wherein
   the content of the compound represented by formula (2) is more than 0.001 parts by mass to 2.5 parts by mass or less relative to 100 parts by mass of the compound represented by formula (1).
[20] A composition comprising
   a compound represented by formula (1): 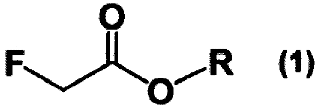
   wherein R is an organic group, and a compound represented by formula (4):

      R-OH (4)
   wherein R is as defined above,
   wherein
   the content of the compound represented by formula (4) is 1.0 mass% or less, or 1.1 to 2.5 mass%.
[21] A composition comprising
   a compound represented by formula (1): 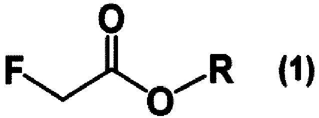
   wherein R is an organic group, and a compound represented by formula (4):

      R-OH (4)
   wherein R is as defined above,
   wherein
   the content of the compound represented by formula (4) is 1.0 part by mass or less, or 1.1 to 2.5 parts by mass relative to 100 parts by mass of the compound represented by formula (1).
[22] A composition comprising
   a compound represented by formula (1): 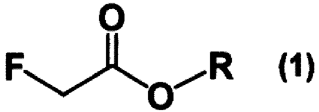
   wherein R is an organic group, and
   2-fluoroacetic acid,
   wherein
   the content of the 2-fluoroacetic acid is 0.9 mass% or less.
[23] A composition comprising
   a compound represented by formula (1): 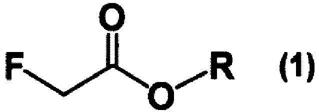
   wherein R is an organic group, and
   2-fluoroacetic acid,
   wherein
   the content of the 2-fluoroacetic acid is 0.9 parts by mass or less relative to 100 parts by mass of the compound represented by formula (1).
[24] A composition comprising
   a compound represented by formula (1): 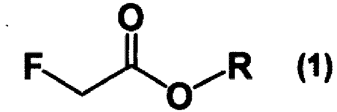
   wherein R is an organic group, and
   a compound represented by formula (5): 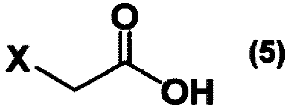
   wherein X is halogen other than fluorine, an alkylsulfonyloxy group, a haloalkylsulfonyloxy group, or an arylsulfonyloxy group, wherein
   the content of the compound represented by formula (5) is 0.001 mass% or less, or 0.002 to 1 mass%.
[25] A composition comprising
   a compound represented by formula (1): 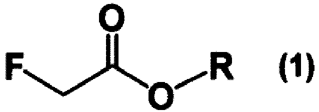
   wherein R is an organic group, and
   a compound represented by formula (5): 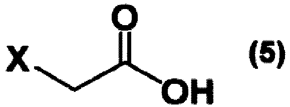
   wherein X is halogen other than fluorine, an alkylsulfonyloxy group, a haloalkylsulfonyloxy group, or an arylsulfonyloxy group,
   wherein
   the content of the compound represented by formula (5) is 0.001 parts by mass or less, or 0.002 to 1 part by mass relative to 100 parts by mass of the compound represented by formula (1).
[26] A composition comprising
   a compound represented by formula (1): 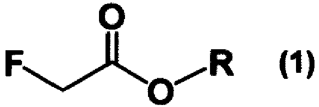
   wherein R is an organic group, and a compound represented by formula (6):

      CH₃COOR (6)
   wherein R is as defined above,
   wherein
   the content of the compound represented by formula (6) is less than 0.01 mass%.
[27] A composition comprising
   a compound represented by formula (1): 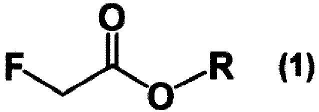
   wherein R is an organic group, and
   a compound represented by formula (6):

      CH₃COOR (6)
   wherein R is as defined above,
   wherein
   the content of the compound represented by formula (6) is less than 0.01 parts by mass relative to 100 parts by mass of the compound represented by formula (1).
[28] A composition comprising
   a compound represented by formula (1): 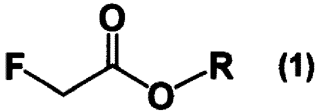
   wherein R is an organic group, and
   a compound represented by formula (7): 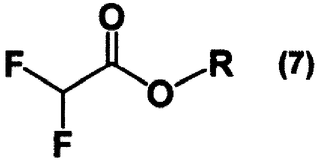
   wherein R is as defined above,
   wherein
   the content of the compound represented by formula (7) is less than 0.01 mass%.
[29] A composition comprising
   a compound represented by formula (1): 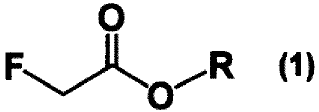
   wherein R is an organic group, and
   a compound represented by formula (7) 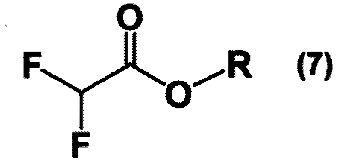
   wherein R is as defined above,
   wherein
   the content of the compound represented by formula (7) is less than 0.01 parts by mass relative to 100 parts by mass of the compound represented by formula (1).
[30] A composition comprising
   a compound represented by formula (1): 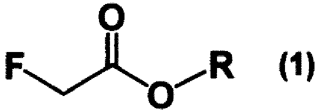
   wherein R is an organic group, and
   a compound represented by formula (8): 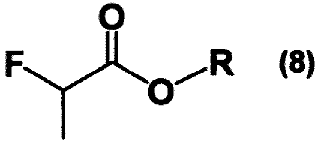
   wherein R is as defined above,
   wherein
   the content of the compound represented by formula (8) is 0.006 to 0.5 mass%.
[31] A composition comprising a compound represented by formula (1): 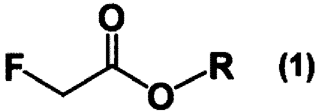
   wherein R is an organic group, and
   a compound represented by formula (8): 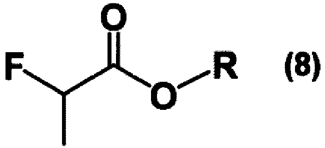
   wherein R is as defined above,
   wherein
   the content of the compound represented by formula (8) is 0.006 to 0.5 parts by mass relative to 100 parts by mass of the compound represented by formula (1).
[32] The composition according to any one of Items 18 to 31, wherein R is a hydrocarbon group optionally having one or more substituents.
[33] The composition according to any one of Items 18 to 32, wherein R is an alkyl group, a fluoroalkyl group, or an aryl group optionally having one or more substituents.
[34] The composition according to any one of Items 18, 19, 24 and 25, wherein X is chlorine or bromine.
[35] A composition comprising at least one inorganic salt selected from the group consisting of a compound represented by formula (1): 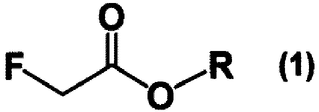 wherein R is an organic group,
   a compound represented by formula (3):

      MFₙ (3)
   wherein M is a cation and n is an integer corresponding to the valence of the cation, and
   a compound represented by formula (3'):

      MClₙ (3')
   wherein M and n are as defined above.
[36] A composition comprising methyl 2-fluoroacetate and ethyl 2-fluoroacetate.

### Advantageous Effects of Invention

According to the present disclosure, a method for producing fluoroacetic acid esters having excellent economic efficiency, quality, productivity, and the like is provided.

### Description of Embodiments

The above summary of the present disclosure is not intended to describe each disclosed embodiment or every implementation of the present disclosure.

The following description of the present disclosure more particularly exemplifies illustrative embodiments.

In several places throughout the present disclosure, guidance is provided through lists of examples, and these examples can be used in various combinations.

In each instance, the described list serves only as a representative group, and should not be interpreted as an exclusive list.

All publications, patents, and patent applications cited herein are incorporated herein by reference in their entirety.

### Terms

Unless otherwise specified, the symbols and abbreviations herein can be understood in the context of the present specification in the meanings commonly used in the art to which the present disclosure belongs.

The term "contains" as used herein is intended to include "consisting essentially of" and "consisting of."

Unless otherwise specified, the steps, treatments, or operations described herein can be performed at room temperature.

The room temperature referred to herein can mean a temperature in the range of 10 to 40°C.

The notation "Cₙ₋ₘ" (where n and m are each a number) used herein means that the number of carbon atoms is n or more and m or less, as is usually understood by persons skilled in the art.

Examples of the "halogen atom" in the present specification include fluorine, chlorine, bromine, and iodine, unless otherwise specified.

The "organic group" in the present specification means a group formed by removing one hydrogen atom from an organic compound.

Examples of the "organic group" include
a hydrocarbon group optionally having one or more substituents,
a non-aromatic heterocyclic group optionally having one or more substituents,
a heteroaryl group optionally having one or more substituents,
a cyano group,
an aldehyde group,
QO-,
QS-,
QCO-,
QS0₂- ,
QOCO-, and
QOS0₂-(wherein Q is independently
a hydrocarbon group optionally having one or more substituents,
a non-aromatic heterocyclic group optionally having one or more substituents, or
a heteroaryl group optionally having one or more substituents).

Examples of the "substituents" include a halogen atom, a cyano group, an amino group, an alkoxy group, and an alkylthio group. Two or more substituents may be the same or different from each other.

Examples of the "hydrocarbon" in the present specification include alkyl, alkenyl, alkynyl, cycloalkyl, cycloalkenyl, cycloalkadienyl, aryl, and aralkyl, unless otherwise specified.

Examples of the "alkyl" in the present specification include linear or branched C₁-₂₀ alkyl groups, such as methyl, ethyl, propyl (e.g., n-propyl and isopropyl), butyl (e.g., n-butyl, isobutyl, sec-butyl, and tert-butyl), pentyl, and hexyl, unless otherwise specified.

Examples of the "alkoxy" in the present specification include linear or branched C₁-₂₀ alkoxy groups, such as methoxy, ethoxy, propoxy (e.g., n-propoxy and isopropoxy), butoxy (e.g., n-butoxy, isobutoxy, sec-butoxy, and tert-butoxy), pentyloxy, and hexyloxy, unless otherwise specified.

Examples of the "alkylthio" in the present specification include linear or branched C₁-₂₀ alkylthio groups, such as methylthio, ethylthio, propylthio (e.g., n-propylthio and isopropylthio), butylthio (e.g., n-butylthio, isobutylthio, sec-butylthio, and tert-butylthio), pentylthio, and hexylthio, unless otherwise specified.

Examples of the "aryloxy" in the present specification include Cc-is aryloxy groups, such as phenyloxy and naphthyloxy, unless otherwise specified.

Examples of the "alkenyl" in the present specification include linear or branched C₂₋₂₀ alkenyl groups, such as vinyl, 1-propen-1-yl, 2-propen-1-yl, isopropenyl, 2-buten-1-yl, 4-penten-1-yl, and 5-hexen-1-yl, unless otherwise specified.

Examples of the "alkynyl" in the present specification include linear or branched C₂-₂₀ alkynyl groups, such as ethynyl, 1-propyn-1-yl, 2-propyn-1-yl, 4-pentyn-1-yl, and 5-hexyn-1-yl, unless otherwise specified.

Examples of the "cycloalkyl" in the present specification include C₃₋₁₀ cycloalkyl groups, such as cyclopropyl, cyclobutyl, cyclopentyl, cyclohexyl, and cycloheptyl, unless otherwise specified.

Examples of the "cycloalkenyl" in the present specification include C₃₋₁₀ cycloalkenyl groups, such as cyclopropenyl, cyclobutenyl, cyclopentenyl, cyclohexenyl, and cycloheptenyl, unless otherwise specified.

Examples of the "cycloalkadienyl" in the present specification include C₄₋₁₀ cycloalkadienyl groups, such as cyclobutadienyl, cyclopentadienyl, cyclohexadienyl, cycloheptadienyl, cyclooctadienyl, cyclononadienyl, and cyclodecadienyl, unless otherwise specified.

The "aryl" in the present specification can be monocyclic, bicyclic, tricyclic, or tetracyclic, unless otherwise specified.

The "aryl" in the present specification can be a C₆₋₁₈ aryl group, unless otherwise specified.

Examples of the "aryl" in the present specification include phenyl, 1-naphthyl, 2-naphthyl, 2-biphenyl, 3-biphenyl, 4-biphenyl, and 2-anthryl, unless otherwise specified.

Examples of the "aralkyl" in the present specification include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylmethyl, 3-biphenylmethyl, and 4-biphenylmethyl, unless otherwise specified.

The "non-aromatic heterocyclic group" in the present specification means a group formed by removing one hydrogen atom from a non-aromatic heterocycle.

The "non-aromatic heterocyclic group" in the present specification can be monocyclic, bicyclic, tricyclic, or tetracyclic, unless otherwise specified.

The "non-aromatic heterocyclic group" in the present specification can be saturated or unsaturated, unless otherwise specified.

The "non-aromatic heterocyclic group" in the present specification can be a 5- to 18-membered non-aromatic heterocyclic group, unless otherwise specified.

Unless otherwise specified, the "non-aromatic heterocyclic group" in the present specification can be, for example, a non-aromatic heterocyclic group containing, in addition to carbon atoms, 1 to 4 heteroatoms selected from oxygen, sulfur, and nitrogen atoms as a ring-constituting atom.

Unless otherwise specified, examples of the "non-aromatic heterocyclic group" in the present specification include tetrahydrofuryl, oxazolidinyl, imidazolinyl (e.g., 1-imidazolinyl, 2-imidazolinyl, and 4-imidazolinyl), aziridinyl (e.g., 1-aziridinyl and 2-aziridinyl), pyrrolidinyl (e.g., 1-pyrrolidinyl, 2-pyrrolidinyl, and 3-pyrrolidinyl), piperidinyl (e.g., 1-piperidinyl, 2-piperidinyl, and 3-piperidinyl), azepanyl (e.g., 1-azepanyl, 2-azepanyl, 3-azepanyl, and 4-azepanyl), azocanyl (e.g., 1-azocanyl, 2-azocanyl, 3-azocanyl, and 4-azocanyl), piperazinyl (e.g., 1,4-piperazin-1-yl and 1,4-piperazin-2-yl), diazepinyl (e.g., 1,4-diazepin-1-yl, 1,4-diazepin-2-yl, 1,4-diazepin-5-yl, and 1,4-diazepin-6-yl), diazocanyl (e.g., 1,4-diazocan-1-yl, 1,4-diazocan-2-yl, 1,4-diazocan-5-yl, 1,4-diazocan-6-yl, 1,5-diazocan-1-yl, 1,5-diazocan-2-yl, and 1,5-diazocan-3-yl), tetrahydropyranyl (e.g., tetrahydrofuran-4-yl), morpholinyl (e.g., 4-morpholinyl), thiomorpholinyl (e.g., 4-thiomorpholinyl), 2-oxazolidinyl, dihydrofuryl, dihydropyranyl, dihydroquinolyl, and the like.

The "heteroaryl" in the present specification can be monocyclic, bicyclic, tricyclic, or tetracyclic, unless otherwise specified.

The "heteroaryl" in the present specification can be, for example, a 5- to 18-membered heteroaryl group.

Unless otherwise specified, the "heteroaryl" in the present specification can be, for example, a heteroaryl group containing, in addition to carbon atoms, 1 to 4 heteroatoms selected from oxygen, sulfur, and nitrogen atoms as a ring-constituting atom.

The "heteroaryl" in the present specification includes "monocyclic heteroaryl" and "aromatic fused heterocyclic group," unless otherwise specified.

Unless otherwise specified, examples of the "monocyclic heteroaryl" in the present specification include pyrrolyl (e.g., 1-pyrrolyl, 2-pyrrolyl, and 3-pyrrolyl), furyl (e.g., 2-furyl and 3-furyl), thienyl (e.g., 2-thienyl and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-3-yl and 1,2,4-triazol-4-yl), oxadiazolyl (e.g., 1,2,4-oxadiazol-3-yl and 1,2,4-oxadiazol-5-yl), thiadiazolyl (e.g., 1,2,4-thiadiazol-3-yl and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), pyrazinyl, and the like.

Unless otherwise specified, examples of the "aromatic fused heterocyclic group" in the present specification include isoindolyl (e.g., 1-isoindolyl, 2-isoindolyl, 3-isoindolyl, 4-isoindolyl, 5-isoindolyl, 6-isoindolyl, and 7-isoindolyl), indolyl (e.g., 1-indolyl, 2-indolyl, 3-indolyl, 4-indolyl, 5-indolyl, 6-indolyl, and 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, and 7-benzo[b]furanyl), benzo[c]furanyl (e.g., 1-benzo[c]furanyl, 4-benzo[c]furanyl, and 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, and 7-benzo[b]thienyl), benzo[c]thienyl (e.g., 1-benzo[c]thienyl, 4-benzo[c]thienyl, and 5-benzo[c]thienyl), indazolyl (e.g., 1-indazolyl, 2-indazolyl, 3-indazolyl, 4-indazolyl, 5-indazolyl, 6-indazolyl, and 7-indazolyl), benzimidazolyl (e.g., 1-benzimidazolyl, 2-benzimidazolyl, 4-benzimidazolyl, and 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, and 1,2-benzisoxazol-7-yl), benzoxazolyl (e.g., 2-benzoxazolyl, 4-benzoxazolyl, 5-benzoxazolyl, 6-benzoxazolyl, and 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, and 1,2-benzisothiazol-7-yl), benzothiazolyl (e.g., 2-benzothiazolyl, 4-benzothiazolyl, 5-benzothiazolyl, 6-benzothiazolyl, and 7-benzothiazolyl), isoquinolyl (e.g., 1-isoquinolyl, 3-isoquinolyl, 4-isoquinolyl, and 5-isoquinolyl), quinolyl (e.g., 2-quinolyl, 3-quinolyl, 4-quinolyl, 5-quinolyl, and 8-quinolyl), cinnolinyl (e.g., 3-cinnolinyl, 4-cinnolinyl, 5-cinnolinyl, 6-cinnolinyl, 7-cinnolinyl, and 8-cinnolinyl), phthalazinyl (e.g., 1-phthalazinyl, 4-phthalazinyl, 5-phthalazinyl, 6-phthalazinyl, 7-phthalazinyl, and 8-phthalazinyl), quinazolinyl (e.g., 2-quinazolinyl, 4-quinazolinyl, 5-quinazolinyl, 6-quinazolinyl, 7-quinazolinyl, and 8-quinazolinyl), quinoxalinyl (e.g., 2-quinoxalinyl, 3-quinoxalinyl, 5-quinoxalinyl, 6-quinoxalinyl, 7-quinoxalinyl, and 8-quinoxalinyl), pyrazolo[1,5-a]pyridyl (e.g., pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, and pyrazolo[1,5-a]pyridin-7-yl), imidazo[1,2-a]pyridyl (e.g., imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl), and the like.

### Method for producing compound represented by formula (1)

In one embodiment, a method for producing a compound represented by formula (1): 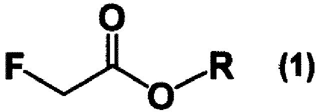 wherein
R is an organic group,
the method comprising
the step (hereinbelow referred to as step (A)) of reacting a compound represented by formula (2): 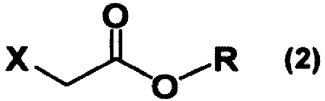
wherein
X is a leaving group other than fluorine, and R is as defined above,
with a compound represented by formula (3):

   MFₙ (3)

   wherein
M is a cation and n is an integer corresponding to the valence of the cation,
wherein
the compound represented by formula (3) is used in an amount of 0.9 moles or less per mole of the compound represented by formula (2) .

### Compound represented by formula (2)

R is preferably a hydrocarbon group optionally having one or more substituents, more preferably alkyl optionally having one or more substituents, or aryl optionally having one or more substituents, even more preferably C₁-₄ alkyl optionally having one or more substituents or C₆-₁₂ aryl optionally having one or more substituents, and particularly preferably C₁-₄ alkyl optionally having one or more substituents.

When R is alkyl optionally having one or more substituents, the one or more substituents are preferably at least one member selected from the group consisting of fluorine, aryl, alkoxy, and aryloxy; more preferably at least one member selected from the group consisting of fluorine, C₆-₁₂ aryl, C₁₋₄ alkoxy, and C₆-₁₂ aryloxy; even more preferably at least one member selected from fluorine and C₆-₁₂ aryl; and particularly preferably fluorine.

When R is aryl optionally having one or more substituents, the one or more substituents are preferably at least one member selected from the group consisting of fluorine, alkyl, and alkoxy; and more preferably at least one member selected from the group consisting of fluorine, C₁-₄ alkyl, and C₁₋₄ alkoxy.

In R, the number of substituents substituted with alkyl or aryl can be selected from the range between 0 and the maximum substitutable number, and varies depending on the type of the substituent. The number of substituents is, for example 0, 1, 2, 3, 4, or 5.

The leaving group represented by X is not limited as long as it is a group that can be removed by the reaction. Examples include halogen other than fluorine, alkylsulfonyloxy, haloalkylsulfonyloxy, and arylsulfonyloxy.

Examples of the alkylsulfonyloxy include C₁₋₆ alkylsulfonyloxy, such as methanesulfonyloxy and ethanesulfonyloxy.

Examples of the haloalkylsulfonyloxy include halo-C₁₋₆ alkylsulfonyloxy, such as trifluoromethanesulfonyloxy and nonafluorobutanesulfonyloxy.

Examples of the arylsulfonyloxy include C₆-₁₈ arylsulfonyloxy such as benzenesulfonyloxy and p-toluenesulfonyloxy.

X is preferably halogen other than fluorine, methanesulfonyloxy, benzenesulfonyloxy, p-toluenesulfonyloxy, trifluoromethanesulfonyloxy, or nonafluorobutanesulfonyloxy; more preferably halogen other than fluorine; even more preferably chlorine or bromine; and particularly preferably chlorine.

### Compound represented by formula (3)

The cation represented by M is not limited, as long as it is a counterion for fluorine ion. Examples include monovalent or divalent cations such as hydrogen ions, alkali metal ions, alkaline earth metal ions, and ammonium ions.

Examples of alkali metal ions include Na⁺, K⁺ and Cs⁺.

Examples of alkaline earth metal ions include Mg²⁺ and Ca²⁺.

Examples of ammonium ions include NH₄⁺, primary ammonium ions, secondary ammonium ions, tertiary ammonium ions, and quaternary ammonium ions.

Examples of primary ammonium ions include primary ammonium ions derived from C₁₋₆ alkyl amines such as methylamine, ethylamine, propylamine (n-propylamine and isopropylamine), and butylamine; and aniline.

Examples of secondary ammonium ions include secondary ammonium ions derived from di-C₁₋₆ alkyl amines such as dimethylamine, diethylamine, ethyl methylamine, and dipropylamine; pyrrolidine; imidazole; piperidine; and morpholine.

Examples of tertiary ammonium ions include tertiary ammonium ions derived from tri-C₁₋₆ alkyl amines such as trimethylamine and triethylamine; pyridine; and quinoline.

Examples of quaternary ammonium ions include tetra C₁₋₆ alkylammonium ions, such as tetramethylammonium ions and tetraethylammonium ions.

M is preferably a hydrogen ion, alkali metal ion, or alkaline earth metal ion; and more preferably, a hydrogen ion or an alkali metal ion.

n can be suitably selected according to the valence of the cation, and is, for example, 1 or 2.

The compound represented by formula (3) is preferably at least one member selected from the group consisting of HF, NaF, KF, CsF, and CaF₂; and more preferably at least one member selected from the group consisting of KF and CsF.

The upper limit of the moisture content of the compound represented by formula (3) is preferably 1.0 mass%, more preferably 0.5 mass%, and even more preferably 0.2 mass%. The lower limit of the moisture content of the compound represented by formula (3) is usually 0.01 mass%. The moisture content of the compound represented by formula (3) is preferably 1.0 mass% or less, and more preferably in the range of 0.01 to 0.5 mass%.

The compound represented by formula (3) is, for example, a powder. The compound represented by formula (3) can also be, for example, a spray-dried product.

The average particle size of the powder can be selected, for example, from the range of 0.5 to 300 pm, preferably the range of 0.5 to 200 pm, more preferably the range of 0.5 to 150 pm, even more preferably the range of 0.5 to 100 pm, and particularly preferably the range of 0.5 to 50 pm. The average particle size can usually be measured by dynamic light scattering, laser diffraction, or centrifugal sedimentation.

The specific surface area of the powder can be selected, for example, from the range of 0.1 to 5.0 m²/g, preferably the range of 0.5 to 5.0 m²/g, and more preferably the range of 1.0 to 5.0 m²/g, from the viewpoint of reaction efficiency. The specific surface area can usually be measured by a transmission method, a gas adsorption method, or a mercury pressure intrusion method.

The amount of the compound represented by formula (3) is 0.9 moles or less, preferably 0.8 moles or less, more preferably 0.7 moles or less, and even more preferably 0.6 moles or less per mole of the compound represented by formula (2). The amount of the compound represented by formula (3) is preferably 0.1 moles or more, more preferably 0.2 moles or more, and even more preferably 0.3 moles or more, per mole of the compound represented by formula (2).

The reaction of step A may be performed in the presence of additives. Examples of additives include catalysts (e.g. phase transfer catalysts). From the viewpoint of preventing impurities from being mixed into the target product, the reaction of step A is preferably performed in the absence of a catalyst (e.g., a phase-transfer catalyst), and even more preferably in the absence of an additive.

The compound represented by formula (2) can be used as a solvent. The reaction of step A may be performed in the presence of one or more further solvents. Examples of further solvents include aliphatic hydrocarbons (e.g., hexane), aromatic hydrocarbons (e.g., toluene and xylene), halogenated hydrocarbons (e.g., dichloromethane and dichloroethane), ethers (e.g., diethyl ether and tetrahydrofuran), ketones (e.g., acetone and methyl ethyl ketone), and nitriles (e.g. acetonitrile). For simplicity of purification and yield, the reaction of step A is preferably performed in the absence of further solvents.

It is preferable that the reaction of step A is substantially performed with the compound represented by formula (2) and the compound represented by formula (3) alone.

The upper limit of the viscosity of the reaction solution in step A at 25°C is preferably 3 Pa·s, 2 Pa·s, or 1 Pa·s.

The lower limit of the viscosity of the reaction solution in step A at 25°C is preferably 0.01 Pa·s.

The viscosity of the reaction solution in step A at 25°C is preferably in the range of 0.01 to 3 Pa·s, 0.01 to 2 Pa·s, or 0.01 to 1 Pa·s. When the viscosity is within such a range, solid precipitation is reduced, the stirring efficiency is increased, and productivity can be improved. The viscosity can be measured using a commonly used viscometer (e.g., SV-10, produced by A&D Corporation).

The lower limit of the reaction temperature in step A is preferably 100°C, more preferably 120°C, and even more preferably 150°C.

The upper limit of the reaction temperature in step A is preferably 250°C, more preferably 240°C, and even more preferably 230°C.

The reaction temperature in step A is preferably in the range of 100 to 250°C, more preferably in the range of 120 to 240°C, and even more preferably in the range of 150 to 230°C.

The lower limit of the reaction time in step A can be preferably 0.5 hours, more preferably 1 hour, even more preferably 2 hours, even more preferably 5 hours, and particularly preferably 10 hours, in terms of conversion.

The upper limit of the reaction time in step A can be preferably 48 hours, more preferably 36 hours, even more preferably 24 hours, and especially preferably 15 hours, in terms of selectivity. The reaction time in step A is preferably within the range of 0.5 to 48 hours, more preferably 1 to 36 hours, even more preferably 2 to 24 hours, and particularly preferably 5 to 15 hours.

It is preferable that the method for producing the compound represented by formula (1) further includes the step of distilling the product obtained in step A (hereinafter sometimes referred to as "step B"). According to the method of the present disclosure, a compound represented by formula (1) can be obtained at a high selectivity even if the product obtained by step A is distilled as is, without being subjected to extraction or other operations.

The distillation of step B is preferably vacuum distillation. The pressure of vacuum distillation can usually be in the range of 0.1 to 98 kPa, preferably in the range of 0.1 to 70 kPa, and more preferably in the range of 0.1 to 40 kPa. The temperature of the vacuum distillation can be higher than the boiling point of the compound represented by formula (1), preferably in the range of 20 to 200°C, and more preferably in the range of 60 to 200°C.

It is preferable that the method for producing the compound represented by formula (1) further includes the step of refining the product obtained in step B (hereinafter sometimes referred to as "step C"). The rectification of step C is preferably performed using a rectification column. Usable examples of the rectification column include plate columns, concentric cylindrical columns, rotation band columns, and packed columns.

The rectification of step C is performed under atmospheric or reduced pressure. The pressure in the rectification is usually in the range of 0.1 to 98 kPa, preferably in the range of 0.1 to 70 kPa, and more preferably in the range of 0.1 to 40 kPa. The temperature in the rectification may be any temperature at which the compound represented by formula (1) can be separated from the compound represented by formula (2) by a difference in boiling point, and is preferably in the range of 20 to 140°C, and more preferably 60 to 140°C.

### Composition

The composition of the present disclosure comprises the compound represented by formula (1) and at least one compound selected from the group consisting of:
the compound represented by formula (2);
the compound represented by formula (4): R-OH (4), wherein R is as defined above;
2-fluoroacetic acid;
the compound represented by formula (5): 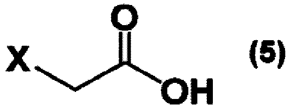
wherein X is as defined above;
acetic acid;
the compound represented by formula (6):

   CH₃COOR (6)
wherein R is as defined above;
the compound represented by formula (7): 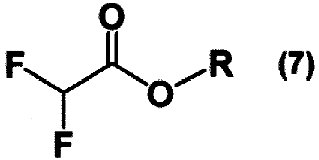
wherein R is as defined above;
the compound represented by formula (8): 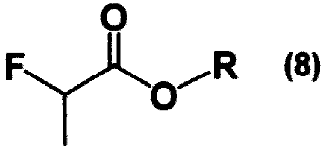
wherein R is as defined above;
the compound represented by formula (9): 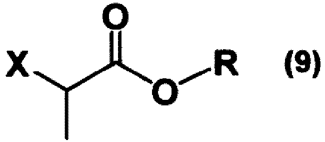 wherein X is as defined above; and at least one inorganic salt (hereinbelow simply referred to as an "inorganic salt") selected from the group consisting of the
compound represented by formula (3) and the compound represented by formula (3'):

   MClₙ (3')
wherein M and n are as defined above.

### Composition a containing compound represented by formula (1) and compound represented by formula (2)

The composition a is not limited as long as it contains the compound represented by formula (1) and the compound represented by formula (2). The compound represented by formula (2) can be, for example, an unreacted starting material in the reaction of step A. The composition a can be, for example, a composition after the reaction of step A, a composition after the distillation of step B, or a composition after the rectification of step C.

In the composition a, the upper limit of the content of the compound represented by formula (2) can be preferably 2.5 mass%, more preferably 2.2 mass%, and even more preferably 2.0 mass%. The upper limit may be 0.001 mass% or the detection limit.

In the composition a, the lower limit of the content of the compound represented by formula (2) can be preferably the detection limit, 0.001 mass%, 0.002 mass%, or 0.003 mass%.

In the composition a, the content of the compound represented by formula (2) can be preferably in the range of more than 0.001 to 2.5 mass% or less, and more preferably in the range of 0.002 to 2.5 mass%.

The upper limit of the content of the compound represented by formula (2) can be preferably 2.5 parts by mass, more preferably 2.3 parts by mass, and even more preferably 2.2 parts by mass, relative to 100 parts by mass of the compound represented by formula (1). The upper limit may be 0.001 mass% or the detection limit.

The lower limit of the content of the compound represented by formula (2) may be preferably the detection limit, and 0.001 parts by mass, 0.002 parts by mass, or 0.003 parts by mass, relative to 100 parts by mass of the compound represented by formula (1).

The content of the compound represented by formula (2) can be preferably in the range of more than 0.001 to 2.5 parts by mass or less, and more preferably in the range of 0.002 to 2.5 parts by mass, relative to 100 parts by mass of the compound represented by formula (1).

The contents of compounds in the composition a can be measured by gas chromatography-mass spectrometry. Gas chromatography conditions can be as follows.

Conditions:
Column: J&W HP-1 produced by Agilent Technologies Ltd., (1.00 pm, 30 m, 0.32 mm ID)
Column oven: 40°C (5 min) -_{>} temperature rise (10°C/min) → 300°C (10 min) or 40°C (5 min) -_{>} temperature rise (7°C/min) -_{>} 280°C (0 min)
Vaporization chamber temperature: 250°C
Detector: Flame ionization detector (FID)

The contents of compounds in compositions b to k described below can be measured in the same manner.

The composition a can be a composition of any of the following:
(a-1) a composition further comprising another component;
(a-2) the composition of (a-1) in which the other component comprises the compound represented by formula (4);
(a-3) the composition of (a-1) or (a-2) in which the other component comprises 2-fluoroacetic acid;
(a-4) the composition of any of (a-1) to (a-3) in which the other component comprises the compound represented by formula (5);
(a-5) the composition of any of (a-1) to (a-4) in which the other component comprises acetic acid;
(a-6) the composition of any of (a-1) to (a-5) in which the other component comprises the compound represented by formula (6);
(a-7) the composition of any of (a-1) to (a-6) in which the other component comprises the compound represented by formula (7);
(a-8) the composition of any of (a-1) to (a-7) in which the other component comprises the compound represented by formula (8);
(a-9) the composition of any of (a-1) to (a-8) in which the other component comprises the compound represented by formula (9); or
(a-10) the composition of any of (a-1) to (a-9) in which the other component comprises an inorganic salt.

In the composition a, the contents of the compound represented by formula (4), 2-fluoroacetic acid, compound represented by formula (5), acetic acid, compound represented by formula (6), compound represented by formula (7), compound represented by formula (8), compound represented by formula (9), and inorganic salt are the same as those described in the sections for compositions b, c, d, e, f, g, h, i, and j below.

### Composition b containing compound represented by formula (1) and compound represented by formula (4)

The composition b is not limited as long as it contains the compound represented by formula (1) and the compound represented by formula (4). The compound represented by formula (4) can be, for example, a side product in the reaction of step A. The composition b can be, for example, a composition after the reaction of step A, a composition after the distillation of step B, or a composition after the rectification of step C.

In the composition b, the upper limit of the content of the compound represented by formula (4) can be preferably 2.5 mass%, more preferably 2.3 mass%, and even more preferably 2.2 mass%. The upper limit may be 1.0 mass%, 0.9 mass%, or the detection limit.

In the composition b, the lower limit of the content of the compound represented by formula (4) can be preferably the detection limit, 0.001 mass%, 1.1 mass%, or 1.2 mass%.

In the composition b, the content of the compound represented by formula (4) can be preferably 1.0 mass% or less and more preferably 0.9 mass% or less, or in the range of 1.1 to 2.5 mass%, and more preferably in the range of 1.2 to 2.5 mass%.

The upper limit of the content of the compound represented by formula (4) can be preferably 2.5 parts by mass, more preferably 2.4 parts by mass, and even more preferably 2.3 parts by mass, relative to 100 parts by mass of the compound represented by formula (1). The upper limit may be 1.0 part by mass, 0.9 parts by mass, or the detection limit.

The lower limit of the content of the compound represented by formula (4) may be preferably the detection limit, and 0.001 parts by mass, 1.1 parts by mass, or 1.2 parts by mass, relative to 100 parts by mass of the compound represented by formula (1).

The content of the compound represented by formula (4) can be preferably 1.0 part by mass or less and more preferably 0.9 parts by mass or less; or in the range of 1.1 to 2.5 parts by mass, and more preferably 1.2 to 2.5 parts by mass, relative to 100 parts by mass of the compound represented by formula (1).

The composition b can be a composition of any of the following:
(b-1) a composition further comprising another component;
(b-2) the composition of (b-1) in which the other component comprises 2-fluoroacetic acid;
(b-3) the composition of (b-1) or (b-2) in which the other component comprises the compound represented by formula (5);
(b-4) the composition of any of (b-1) to (b-3)in which the other component comprises acetic acid;
(b-5) the composition of any of (b-1) to (b-4) in which the other component comprises the compound represented by formula (6);
(b-6) the composition of any of (b-1) to (b-5) in which the other component comprises the compound represented by formula (7);
(b-7) the composition of any of (b-1) to (b-6) in which the other component comprises the compound represented by formula (8);
(b-8) the composition of any of (b-1) to (b-7) in which the other component comprises the compound represented by formula (9); or
(b-9) the composition of any of (b-1) to (b-8) in which the other component comprises an inorganic salt.

In the composition b, the contents of the 2-fluoroacetic acid, compound represented by formula (5), acetic acid, compound represented by formula (6), compound represented by formula (7), compound represented by formula (8), compound represented by formula (9), and inorganic salt are the same as those described in the sections for compositions c, d, e, f, g, h, i, and j below.

### Composition c containing compound represented by formula (1) and 2-fluoroacetic acid

The composition c is not limited as long as it contains the compound represented by formula (1) and 2-fluoroacetic acid. The 2-fluoroacetic acid can be, for example, a side product in the reaction of step A, specifically, the hydrolysate of the compound represented by formula (1).

The composition c can be, for example, a composition after the reaction of step A, a composition after the distillation of step B, or a composition after the rectification of step C.

In the composition c, the upper limit of the content of the 2-fluoroacetic acid can be preferably 0.9 mass%, more preferably 0.8 mass%, even more preferably 0.7 mass%, and particularly preferably 0.6 mass%.

In the composition c, the lower limit of the content of the 2-fluoroacetic acid can be usually the detection limit or 0.001 mass%.

In the composition c, the content of the 2-fluoroacetic acid can be preferably 0.9 mass% or less, and more preferably in the range of 0.001 to 0.8 mass%

The upper limit of the content of the 2-fluoroacetic acid can be preferably 0.9 parts by mass, more preferably 0.8 parts by mass, even more preferably 0.7 parts by mass, and particularly preferably 0.6 parts by mass, relative to 100 parts by mass of the compound represented by formula (1).

The lower limit of the content of the 2-fluoroacetic acid can be usually the detection limit, or 0.001 parts by mass, relative to 100 parts by mass of the compound represented by formula (1).

The content of the 2-fluoroacetic acid can be preferably 0.9 parts by mass or less and more preferably in the range of 0.001 to 0.8 parts by mass, relative to 100 parts by mass of the compound represented by formula (1).

The composition c can be a composition of any of the following:
(c-1) a composition further comprising another component;
(c-2) the composition of (c-1) in which the other component comprises the compound represented by formula (5);
(c-3) the composition of (c-1) or (c-2) in which the other component comprises acetic acid;
(c-4) the composition of any of (c-1) to (c-3) in which the other component comprises the compound represented by formula (6);
(c-5) the composition of any of (c-1) to (c-4) in which the other component comprises the compound represented by formula (7);
(c-6) the composition of any of (c-1) to (c-5) in which the other component comprises the compound represented by formula (8);
(c-7) the composition of any of (c-1) to (c-6) in which the other component comprises the compound represented by formula (9); or
(c-8) the composition of any of (c-1) to (c-7) in which the other component comprises an inorganic salt.

In the composition c, the contents of the compound represented by formula (5), acetic acid, compound represented by formula (6), compound represented by formula (7), compound represented by formula (8), compound represented by formula (9), and inorganic salt are the same as those described in the sections for compositions d, e, f, g, h, i, and j below.

### Composition d containing compound represented by formula (1) and compound represented by formula (5)

The composition d is not limited as long as it contains the compound represented by formula (1) and the compound represented by formula (5). The compound represented by formula (5) can be, for example, a side product in the reaction of step A, specifically, the hydrolysate of the compound represented by formula (2). The composition d can be, for example, a composition after the reaction of step A, a composition after the distillation of step B, or a composition after the rectification of step C.

In the composition d, the upper limit of the content of the compound represented by formula (5) can be preferably 1 mass%, more preferably 0.5 mass%, and even more preferably 0.1 mass%. The upper limit may be 0.001 mass% or the detection limit.

In the composition d, the lower limit of the content of the compound represented by formula (5) can be preferably the detection limit, 0.002 mass%, 0.003 mass%, or 0.004 mass%.

In the composition d, the content of the compound represented by formula (5) can be preferably 0.001 mass% or less or in the range of 0.002 to 1 mass%.

The upper limit of the content of the compound represented by formula (5) can be preferably 1 part by mass, more preferably 0.5 parts by mass, and even more preferably 0.1 parts by mass, relative to 100 parts by mass of the compound represented by formula (1). The upper limit may be 0.001 parts by mass or the detection limit.

The lower limit of the content of the compound represented by formula (5) may be preferably the detection limit, and 0.002 parts by mass, 0.003 parts by mass, or 0.004 parts by mass, relative to 100 parts by mass of the compound represented by formula (1).

The content of the compound represented by formula (5) can be preferably 0.001 parts by mass or less or in the range of 0.002 to 1 part by mass, relative to 100 parts by mass of the compound represented by formula (1).

The composition d can be a composition of any of the following:
(d-1) a composition further comprising another component;
(d-2) the composition of (d-1) in which the other component comprises acetic acid;
(d-3) the composition of (d-1) or (d-2) in which the other component comprises the compound represented by formula (6);
(d-4) the composition of any of (d-1) to (d-3) in which the other component comprises the compound represented by formula (7);
(d-5) the composition of any of (d-1) to (d-4) in which the other component comprises the compound represented by formula (8);
(d-6) the composition of any of (d-1) to (d-5) in which the other component comprises the compound represented by formula (9); or
(d-7) the composition of any of (d-1) to (d-6) in which the other component comprises an inorganic salt.

In the composition d, the contents of the acetic acid, compound represented by formula (6), compound represented by formula (7), compound represented by formula (8), compound represented by formula (9), and inorganic salt are the same as those described in the sections for compositions e, f, g, h, i, and j below.

### Composition e containing compound represented by formula (1) and acetic acid

The composition e is not limited as long as it contains the compound represented by formula (1) and acetic acid. The acetic acid can be, for example, a side product in the reaction of step A. The composition e can be, for example, a composition after the reaction of step A, a composition after the distillation of step B, or a composition after the rectification of step C.

In the composition e, the upper limit of the content of acetic acid can be preferably 2.5 mass%, more preferably 1.5 mass%, and even more preferably 1.0 mass%.

In the composition e, the lower limit of the content of the acetic acid can be usually the detection limit or 0.001 mass%.

In the composition e, the content of the acetic acid can be preferably 2.5 mass% or less, and more preferably in the range of 0.001 to 1.0 mass%.

The upper limit of the content of the acetic acid can be preferably 2.5 parts by mass, more preferably 1.5 parts by mass, and even more preferably 1.0 part by mass, relative to 100 parts by mass of the compound represented by formula (1).

The lower limit of the content of the acetic acid can be usually the detection limit, or 0.001 parts by mass, relative to 100 parts by mass of the compound represented by formula (1).

The content of the acetic acid can be preferably 2.5 parts by mass or less and more preferably in the range of 0.001 to 1.0 part by mass, relative to 100 parts by mass of the compound represented by formula (1).

The composition e can be a composition of any of the following:
(e-1) a composition further comprising another component;
(e-2) the composition of (e-1) in which the other component comprises the compound represented by formula (6);
(e-3) the composition of (e-1) or (e-2) in which the other component comprises the compound represented by formula (7);
(e-4) the composition of any of (e-1) to (e-3) in which the other component comprises the compound represented by formula (8);
(e-5) the composition of any of (e-1) to (e-4) in which the other component comprises the compound represented by formula (9); or
(e-6) the composition of any of (e-1) to (e-5) in which the other component comprises an inorganic salt.

In the composition e, the contents of the compound represented by formula (6), compound represented by formula (7), compound represented by formula (8), compound represented by formula (9), and inorganic salt are the same as those described in the sections for compositions f, g, h, i, and j below.

### Composition f containing compound represented by formula (1) and compound represented by formula (6)

The composition f is not limited as long as it contains the compound represented by formula (1) and the compound represented by formula (6). The compound represented by formula (6) can be a side product in the reaction of step A. The composition f can be, for example, a composition after the reaction of step A, a composition after the distillation of step B, or a composition after the rectification of step C.

In the composition f, the upper limit of the content of the compound represented by formula (6) can be preferably 0.01 mass%, more preferably 0.009 mass%, and even more preferably 0.008 mass%.

In the composition f, the lower limit of the content of the compound represented by formula (6) can be usually the detection limit or 0.001 mass%.

In the composition f, the content of the compound represented by formula (6) can be preferably less than 0.01 mass%, and more preferably in the range of 0.001 to 0.009 mass%.

The upper limit of the content of the compound represented by formula (6) can be preferably 0.01 parts by mass, more preferably 0.009 parts by mass, and even more preferably 0.008 parts by mass, relative to 100 parts by mass of the compound represented by formula (1).

The lower limit of the content of the compound represented by formula (6) may be usually the detection limit, or 0.001 parts by mass relative to 100 parts by mass of the compound represented by formula (1).

The content of the compound represented by formula (6) can be preferably less than 0.01 parts, and more preferably in the range of 0.001 to 0.009 parts by mass, relative to 100 parts by mass of the compound represented by formula (1).

The composition f can be a composition of any of the following:
(f-1) a composition further comprising another component;
(f-2) the composition of (f-1) in which the other component comprises the compound represented by formula (7);
(f-3) the composition of (f-1) or (f-2) in which the other component comprises the compound represented by formula (8);
(f-4) the composition of any of (f-1) to (f-3) in which the other component comprises the compound represented by formula (9); or
(f-5) the composition of any of (f-1) to (f-4) in which the other component comprises an inorganic salt.

In the composition f, the contents of the compound represented by formula (7), compound represented by formula (8), compound represented by formula (9), and inorganic salt are the same as those described in the sections for compositions g, h, i, and j below.

### Composition g containing compound represented by formula (1) and compound represented by formula (7)

The composition g is not limited as long as it contains the compound represented by formula (1) and the compound represented by formula (7). The compound represented by formula (7) can be a side product in the reaction of step A. The composition g can be, for example, a composition after the reaction of step A, a composition after the distillation of step B, or a composition after the rectification of step C.

In the composition g, the upper limit of the content of the compound represented by formula (7) can be preferably 0.01 mass%, more preferably 0.009 mass%, and even more preferably 0.008 mass%.

In the composition g, the lower limit of the content of the compound represented by formula (7) can be usually the detection limit or 0.001 mass%.

In the composition g, the content of the compound represented by formula (7) can be preferably less than 0.01 mass%, and more preferably in the range of 0.001 to 0.009 mass%.

The upper limit of the content of the compound represented by formula (7) can be preferably 0.01 parts by mass, more preferably 0.009 parts by mass, and even more preferably 0.008 parts by mass, relative to 100 parts by mass of the compound represented by formula (1).

The lower limit of the content of the compound represented by formula (7) may be usually the detection limit, or 0.001 parts by mass, relative to 100 parts by mass of the compound represented by formula (1).

The content of the compound represented by formula (7) can be preferably less than 0.01 parts by mass, and more preferably in the range of 0.001 to 0.009 parts by mass, relative to 100 parts by mass of the compound represented by formula (1).

The composition g can be a composition of any of the following:
(g-1) a composition further comprising another component;
(g-2) the composition of (g-1) in which the other component comprises the compound represented by formula (8);
(g-3) the composition of (g-1) or (g-2) in which the other component comprises the compound represented by formula (9); or
(g-4) the composition of any of (g-1) to (g-3) in which the other component comprises an inorganic salt.

In the composition g, the contents of the compound represented by formula (8), compound represented by formula (9), and inorganic salt are the same as those described in the sections for compositions h, i, and j below.

### Composition h containing compound represented by formula (1) and compound represented by formula (8)

The composition h is not limited as long as it contains the compound represented by formula (1) and the compound represented by formula (8). The compound represented by formula (8) can be a side product in the reaction of step A. The composition h can be, for example, a composition after the reaction of step A, a composition after the distillation of step B, or a composition after the rectification of step C.

In the composition h, the upper limit of the content of the compound represented by formula (8) can be preferably 0.5 mass%, and more preferably 0.1 mass%. The upper limit may be 0.005 mass%, 0.004 mass%, or detection limit.

In the composition h, the lower limit of the content of the compound represented by formula (8) can be usually 0.006 or 0.007 mass%.

In the composition h, the content of the compound represented by formula (8) can be preferably in the range of 0.006 to 0.5 mass%, and more preferably in the range of 0.007 to 0.1 mass%.

The upper limit of the content of the compound represented by formula (8) can be preferably 0.5 parts by mass, and more preferably 0.1 parts by mass, relative to 100 parts by mass of the compound represented by formula (1); and may be 0.005 parts by mass, 0.004 parts by mass, or the detection limit.

The lower limit of the content of the compound represented by formula (8) can be usually 0.006 or 0.007 parts by mass, relative to 100 parts by mass of the compound represented by formula (1).

The content of the compound represented by formula (8) can be preferably in the range of 0.006 to 0.5 parts by mass, and more preferably 0.007 to 0.1 parts by mass, relative to 100 parts by mass of the compound represented by formula (1).

The composition h can be a composition of any of the following:
(h-1) a composition further comprising another component;
(h-2) the composition of (h-1) in which the other component comprises the compound represented by formula (9); or
(h-3) the composition of (h-1) or (h-2) in which the other component comprises an inorganic salt.

In the composition h, the contents of the compound represented by formula (9) and inorganic salt are the same as those described in the sections for compositions i and j below.

### Composition i containing compound represented by formula (1) and compound represented by formula (9)

The composition i is not limited as long as it contains the compound represented by formula (1) and the compound represented by formula (9). The compound represented by formula (9) can be a side product in the reaction of step A. The composition i can be, for example, a composition after the reaction of step A, a composition after the distillation of step B, or a composition after the rectification of step C.

In the composition i, the upper limit of the content of the compound represented by formula (9) can be preferably 1.0 mass%, more preferably 0.9 mass%, and even more preferably 0.8 mass%.

In the composition i, the lower limit of the content of the compound represented by formula (9) can be usually the detection limit or 0.001 mass%.

In the composition i, the content of the compound represented by formula (9) can be preferably in the range of more than the detection limit to 1.0 mass% or less, and more preferably in the range of 0.001 to 0.9 mass%.

The upper limit of the content of the compound represented by formula (9) can be preferably 1.0 part by mass, more preferably 0.9 parts by mass, and even more preferably 0.8 parts by mass, relative to 100 parts by mass of the compound represented by formula (1).

The lower limit of the content of the compound represented by formula (9) may be usually the detection limit or 0.001 parts by mass, relative to 100 parts by mass of the compound represented by formula (1).

The content of the compound represented by formula (9) can be preferably more than the detection limit and 1.0 part by mass or less, and more preferably 0.001 to 0.9 parts by mass, relative to 100 parts by mass of the compound represented by formula (1).

The composition i can be a composition further comprising an inorganic salt. In the composition i, the content of the inorganic salt is the same as that described in the section for a composition j below.

### Composition j containing compound represented by formula (1), and at least one member selected from the group consisting of compound represented by formula (3) and compound represented by formula (3')

The composition j is not limited as long as it contains the compound represented by formula (1), and at least one inorganic salt selected from the group consisting of the compound represented by formula (3) and the compound represented by formula (3'):

MClₙ (3')

wherein M and n are as defined above. The compound represented by formula (3) can be an unreacted starting material in the reaction of step A. The compound represented by formula (3') can be, for example, a side product. The composition j can be, for example, a composition after the reaction of step A, a composition after the distillation of step B, or a composition after the rectification of step C.

In the composition j, the upper limit of the content of the inorganic salt can be preferably 0.01 mass%. In the composition j, the lower limit of the content of the inorganic salt can be usually the detection limit. In the composition j, the content of the inorganic salt can be preferably in the range of more than the detection limit to 0.01 mass% or less.

The upper limit of the content of the inorganic salt can be preferably 0.01 parts by mass, relative to 100 parts by mass of the compound represented by formula (1).

The lower limit of the content of the inorganic salt can be usually the detection limit.

The content of the inorganic salt can be preferably in the range of more than the detection limit to 0.01 parts by mass or less, relative to 100 parts by mass of the compound represented by formula (1).

The content of the inorganic salt in the composition j can be measured by ion chromatography-mass spectrometry. The ion chromatography conditions can be as follows. Conditions
Column: produced by Thermo Fisher Scientific
Dionex IonPac AS4A-SC, 4 x 250 mm
Column oven: 45°C
Eluent: 4 mM sodium carbonate aqueous solution
Eluent flow rate: 0.8 ml/min
Detector: conductometer

### Composition k containing methyl 2-fluoroacetate and ethyl 2-fluoroacetate

The composition k is not limited as long as it contains methyl 2-fluoroacetate and ethyl 2-fluoroacetate.

In the composition k, the upper limit of the content of the ethyl 2-fluoroacetate can be preferably 0.019 mass%, more preferably 0.015 mass%, and even more preferably 0.010 mass%.

In the composition k, the lower limit of the content of the ethyl 2-fluoroacetate can be usually the detection limit or 0.001 mass%.

In the composition k, the content of the ethyl 2-fluoroacetate can be preferably 0.019 mass% or less and more preferably in the range of 0.001 to 0.015 mass%.

The upper limit of the content of the ethyl 2-fluoroacetate can be preferably 0.019 parts by mass, more preferably 0.015 parts by mass, and even more preferably 0.010 parts by mass, relative to 100 parts by mass of the methyl 2-fluoroacetate.

The lower limit of the content of the ethyl 2-fluoroacetate can be usually the detection limit or 0.001 parts by mass, relative to 100 parts by mass of the methyl 2-fluoroacetate.

The content of the ethyl 2-fluoroacetate can be preferably 0.019 parts by mass or less and more preferably in the range of 0.001 to 0.015 parts by mass, relative to 100 parts by mass of the methyl 2-fluoroacetate.

The composition k can be a composition of any of the following:
(k-1) a composition further comprising another component;
(k-2) the composition of (k-1) in which the other component comprises the compound represented by formula (2);
(k-3) the composition of (k-1) or (k-2) in which the other component comprises the compound represented by formula (4);
(k-4) the composition of any of (k-1) to (k-3) in which the other component comprises 2-fluoroacetic acid;
(k-5) the composition of any of (k-1) to (k-4) in which the other component comprises the compound represented by formula (5);
(k-6) the composition of any of (k-1) to (k-5) in which the other component comprises acetic acid;
(k-7) the composition of any of (k-1) to (k-6) in which the other component comprises the compound represented by formula (6);
(k-8) the composition of any of (k-1) to (k-7) in which the other component comprises the compound represented by formula (7);
(k-9) the composition of any of (k-1) to (k-7) in which the other component comprises the compound represented by formula (8);
(k-10) the composition of any of (k-1) to (k-9) in which the other component comprises the compound represented by formula (9); or
(k-11) the composition of any of (k-1) to (k-10) in which the other component comprises an inorganic salt.

In the composition k, the contents of the compound represented by formula (2), compound represented by formula (4), 2-fluoroacetic acid, compound represented by formula (5), acetic acid, compound represented by formula (6), compound represented by formula (7), compound represented by formula (8), compound represented by formula (9), and inorganic salt are the same as those described in the sections for compositions a, b, c, d, e, f, g, h, i, and j above.

### Examples

One embodiment of the present disclosure is described in more detail below by way of the Examples; however, the disclosure is not limited thereto.

The viscosity of a suspension comprising methyl chloroacetate and 1.0 molar equivalent of potassium fluoride (corresponding to the reaction solution in the Comparative Example) and a suspension comprising methyl chloroacetate and 0.5 molar equivalent of potassium fluoride (corresponding to the reaction solution in the Example) at 25°C was measured using a viscometer (SV-10, produced by A&D Company Ltd.). The results are shown in Table 1.

**Table 1**

| | Viscosity (25 °C) |
|---|---|
| Methyl chloroacetate + potassium fluoride (1.0 molar equivalent) | 5 Pa-s |
| Methyl chloroacetate + potassium fluoride (0.5 molar equivalent) | 0.6 Pa-s |

The results showed that the use of 0.5 molar equivalent of potassium fluoride could reduce viscosity (reduce solid precipitation), provide excellent stirring efficiency, and improve productivity as compared to the use of 1.0 molar equivalent of potassium fluoride.

Potassium fluoride (30.7 g, 0.53 mol) and methyl chloroacetate (116 g, 1.07 mol) were placed in an autoclave, and then the autoclave was sealed. With stirring, the internal temperature was raised to 208°C and the reaction was performed for 12 hours. The conversion and selectivity at 2, 5, and 12 hours after the start of the reaction are shown in Table 2.

**Table 2**

| Reaction time (hour) | Conversion (%) | Selectivity (%) |
|---|---|---|
| 2 | 46 | 94 |
| 5 | 83 | 93 |
| 12 | 90 | 90 |

After cooling, the crude solution was extracted by vacuum distillation. Rectification was then performed to isolate methyl fluoroacetate. The formulation of the composition immediately after reaction, after vacuum distillation, and after rectification is shown in Table 3.

**Table 3**

| | Immediately after reaction | After vacuum distillation | After rectification Example 1 | After rectification Example 2 |
|---|---|---|---|---|
| MFAc | 39.8% | 33.8% | 99.1% | 95.3% |
| MCAc | 54.2% | 63.7% | 0.8225% | 1.9231% |
| KF | 0.3% | 0.1% | 0.506 ppm | - |
| KCl | 0.4% | 0.1% | 0.514 ppm | - |
| MeOH | 1.0% | 0.8% | 0.0177% | 2.1229% |
| FAA | 0.5% | 0.5% | - | 0.5196% |
| CAA | 0.4% | 0.3% | - | 0.0522% |
| Acetic acid | 0.1% | 0.1% | - | - |
| Methyl acetate | 0.007% | 0.007% | 0.005% | 0.001% |
| Methyl 2,2-difluoro acetate | 0.007% | 0.007% | 0.003% | 0.005% |
| CH₃CHFC0₂Me | 0.006% | 0.09% | 0.0483% | 0.0542% |
| CH₃CHClCO₂Me | 0.6% | 0.5% | 0.0219% | 0.0333% |

In the table, "%" means "mass%" and "-" means "below detection limit."

### Abbreviations

MFAc: Methyl fluoroacetate
MCAc: Methyl chloroacetate
MeOH: Methanol
FAA: Fluoroacetic acid
CAA: Chloroacetic acid

### Composition analysis method

The content of each of the components other than KF and KCl was analyzed by gas chromatography (under the following conditions). Conditions
Column: J&W HP-1 produced by Agilent Technologies Ltd (1.00 pm, 30 m, 0.32 mm ID)
Column oven: 40°C (5 min) → temperature rise (10°C/min) -_{>} 300°C (10 min) or 40°C (5 min) -_{>} temperature rise (7°C/min) -_{>} 280°C (0 min)
Vaporization chamber temperature: 250°C
Detector: Flame ionization detector (FID)

The content of KF and the content of KCl were respectively analyzed as F ions and Cl ions by ion chromatography (under the following conditions). Conditions
Column: produced by Thermo Fisher Scientific
Dionex IonPac AS4A-SC 4 x 250 mm
Column oven: 45°C
Eluent: 4 mM sodium carbonate aqueous solution
Eluent flow rate: 0.8 ml/min
Detector: conductometer

## Claims

1. A method for producing a compound represented by formula (1) : 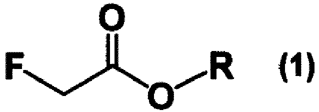 wherein
R is an organic group,
the method comprising
the step of reacting a compound represented by formula (2): 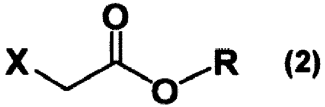
wherein
X is a leaving group other than fluorine, and R is as defined above,
with a compound represented by formula (3):
MFₙ (3)
wherein
M is a cation and n is an integer corresponding to the valence of the cation,
wherein
the compound represented by formula (3) is used in an amount of 0.9 moles or less per mole of the compound represented by formula (2).

2. The production method according to claim 1, wherein the reaction is substantially performed with the compound represented by formula (2) and the compound represented by formula (3) alone.

3. The production method according to claim 1 or 2, wherein the compound represented by formula (3) is used in an amount of 0.8 moles or less per mole of the compound represented by formula (2).

4. The production method according to any one of claims 1 to 3, wherein the compound represented by formula (3) is used in an amount of 0.7 moles or less per mole of the compound represented by formula (2).

5. The production method according to any one of claims 1 to 4, wherein the compound represented by formula (3) is used in an amount of 0.6 moles or less per mole of the compound represented by formula (2).

6. The production method according to any one of claims 1 to 5, wherein the reaction is performed at 100 to 250°C.

7. The production method according to any one of claims 1 to 6, comprising the step of distilling a product obtained by the above step.

8. The production method according to any one of claims 1 to 7, wherein R is a hydrocarbon group optionally having one or more substituents.

9. The production method according to any one of claims 1 to 8, wherein R is an alkyl group, a fluoroalkyl group, or an aryl group optionally having one or more substituents.

10. The production method according to any one of claims 1 to 9, wherein X is halogen other than fluorine, an alkylsulfonyloxy group, a haloalkylsulfonyloxy group, or an arylsulfonyloxy group.

11. The production method according to any one of claims 1 to 10, wherein X is chlorine or bromine.

12. The production method according to any one of claims 1 to 11, wherein X is chlorine.

13. The production method according to any one of claims 1 to 12, wherein M is a hydrogen ion, an alkali metal ion, an alkaline earth metal ion, or an ammonium ion.

14. The production method according to any one of claims 1 to 13, wherein M is a hydrogen ion or an alkali metal ion.

15. The production method according to any one of claims 1 to 13, wherein the compound represented by formula (3) is at least one member selected from HF, NaF, KF, CsF, and CaF₂.

16. The production method according to any one of claims 1 to 15, wherein the compound represented by formula (3) is at least one member selected from KF and CsF.

17. The production method according to any one of claims 1 to 16, wherein the compound represented by formula (3) has a moisture content of 1.0 mass% or less.

18. A composition comprising
a compound represented by formula (1): 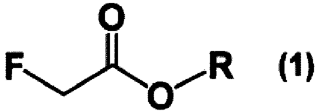
wherein R is an organic group, and
a compound represented by formula (2): 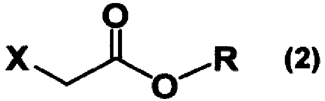
wherein X is halogen other than fluorine, an alkylsulfonyloxy group, a haloalkylsulfonyloxy group, or an arylsulfonyloxy group, and R is an organic group,
wherein the content of the compound represented by formula (2) is more
than 0.001 mass% to 2.5 mass% or less.

19. A composition comprising
a compound represented by formula (1): 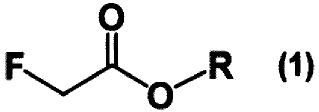
wherein R is an organic group, and
a compound represented by formula (2): 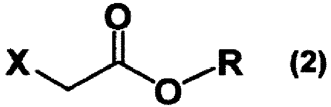
wherein X is halogen other than fluorine, an alkylsulfonyloxy group, a haloalkylsulfonyloxy group, or an arylsulfonyloxy group, and R is an organic group,
wherein
the content of the compound represented by formula (2) is more
than 0.001 parts by mass to 2.5 parts by mass or less relative to 100 parts by mass of the compound represented by formula (1).

20. A composition comprising
a compound represented by formula (1): 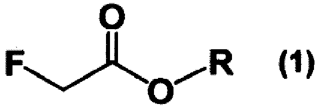
wherein R is an organic group, and
a compound represented by formula (4):
R-OH (4)
wherein R is as defined above,
wherein
the content of the compound represented by formula (4) is 1.0 mass% or less, or 1.1 to 2.5 mass%.

21. A composition comprising
a compound represented by formula (1): 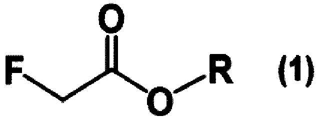
wherein R is an organic group, and
a compound represented by formula (4):
R-OH (4)
wherein R is as defined above,
wherein
the content of the compound represented by formula (4) is 1.0 part by mass or less, or 1.1 to 2.5 parts by mass relative to 100 parts by mass of the compound represented by formula (1).

22. A composition comprising
a compound represented by formula (1): 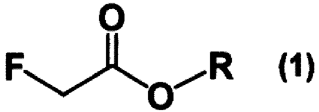
wherein R is an organic group, and
2-fluoroacetic acid,
wherein
the content of the 2-fluoroacetic acid is 0.9 mass% or less.

23. A composition comprising
a compound represented by formula (1): 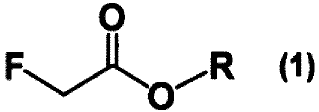
wherein R is an organic group, and
2-fluoroacetic acid,
wherein
the content of the 2-fluoroacetic acid is 0.9 parts by mass or less relative to 100 parts by mass of the compound represented by formula (1).

24. A composition comprising
a compound represented by formula (1): 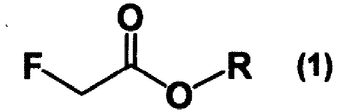
wherein R is an organic group, and
a compound represented by formula (5): 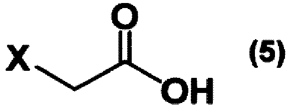
wherein X is halogen other than fluorine, an alkylsulfonyloxy group, a haloalkylsulfonyloxy group, or an arylsulfonyloxy group,
wherein
the content of the compound represented by formula (5) is 0.001 mass% or less, or 0.002 to 1 mass%.

25. A composition comprising
a compound represented by formula (1): 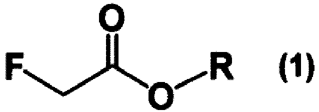
wherein R is an organic group, and
a compound represented by formula (5): 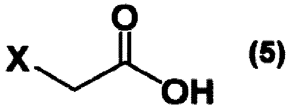
wherein X is halogen other than fluorine, an alkylsulfonyloxy group, a haloalkylsulfonyloxy group, or an arylsulfonyloxy group,
wherein
the content of the compound represented by formula (5) is 0.001 parts by mass or less, or 0.002 to 1 part by mass relative to 100 parts by mass of the compound represented by formula (1).

26. A composition comprising
a compound represented by formula (1): 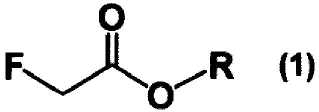
wherein R is an organic group, and
a compound represented by formula (6):
CH₃COOR (6)
wherein R is as defined above,
wherein
the content of the compound represented by formula (6) is less than 0.01 mass%.

27. A composition comprising
a compound represented by formula (1): 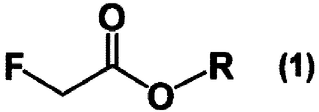
wherein R is an organic group, and
a compound represented by formula (6):
CH₃COOR (6)
wherein R is as defined above,
wherein
the content of the compound represented by formula (6) is less than 0.01 parts by mass relative to 100 parts by mass of the compound represented by formula (1).

28. A composition comprising
a compound represented by formula (1): 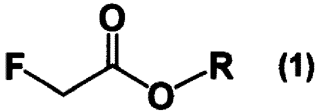
wherein R is an organic group, and
a compound represented by formula (7): 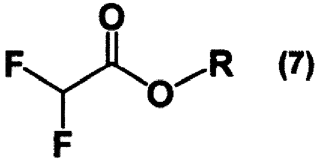
wherein R is as defined above,
wherein
the content of the compound represented by formula (7) is less than 0.01 mass%.

29. A composition comprising
a compound represented by formula (1): 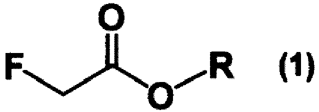
wherein R is an organic group, and
a compound represented by formula (7) 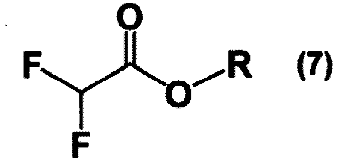
wherein R is as defined above,
wherein
the content of the compound represented by formula (7) is less than 0.01 parts by mass relative to 100 parts by mass of the compound represented by formula (1).

30. A composition comprising
a compound represented by formula (1): 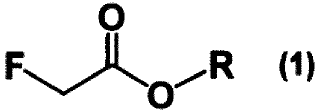
wherein R is an organic group, and
a compound represented by formula (8): 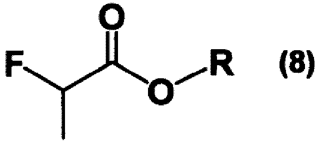
wherein R is as defined above,
wherein
the content of the compound represented by formula (8) is 0.006 to 0.5 mass%.

31. A composition comprising a compound represented by formula (1): 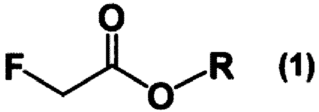
wherein R is an organic group, and
a compound represented by formula (8): 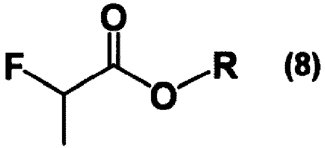
wherein R is as defined above,
wherein
the content of the compound represented by formula (8) is 0.006 to 0.5 parts by mass relative to 100 parts by mass of the compound represented by formula (1).

32. The composition according to any one of claims 18 to 31, wherein R is a hydrocarbon group optionally having one or more substituents.

33. The composition according to any one of claims 18 to 32, wherein R is an alkyl group, a fluoroalkyl group, or an aryl group optionally having one or more substituents.

34. The composition according to any one of claims 18, 19, 24 and 25, wherein X is chlorine or bromine.

35. A composition comprising a compound represented by formula (1): 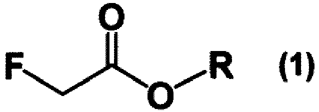
wherein R is an organic group, and
at least one inorganic salt selected from the group consisting of
a compound represented by formula (3):
MFₙ (3)
wherein M is a cation and n is an integer corresponding to the valence of the cation, and
a compound represented by formula (3'):
MClₙ (3')
wherein M and n are as defined above.

36. A composition comprising methyl 2-fluoroacetate and ethyl 2-fluoroacetate.
